# EUROPEAN PATENT APPLICATION

(11) **EP 2 201 958 A1**
(43) Date of publication of application: **30.06.2010**
(21) Application number: 08022332.4
(22) Date of filing: 23.12.2008
(51) Int. Cl.: A61K 38/16

(54) **Method for the preparation of pro-apoptotic protein carrying systems and their uses**

(71) Applicant: Zongoli, Fabrizio, 72100 Brindisi (BR) (IT)
(72) Inventor: Zongoli, Fabrizio, 72100 Brindisi (BR) (IT)
(74) Representative: Trupiano, Federica

(57) **Abstract**

The present invention concerns a method for the preparation of suitable carrier systems for pro-apoptotic proteins and accessory proteins suitable for cell recognition which comprises the development and preparation of lipidic particles able to express pro-apoptotic proteins and accessory proteins suitable for cell recognition.

## Description

### STATE OF THE ART

The term neoplasia, which literally means new formation, is used as a synonym of the term tumour, but unlike the latter it is not much used to refer to the external appearance of the mass but rather to the cellular content of the same, which consists in fact of "new formation" cells.

The incidence of tumoural pathologies has increased over time, and likewise their heterogeneity, consequently the term neoplasia is also used to define a class of diseases characterised by an uncontrolled reproduction of cells in the organism, which no longer respond to the physiological cellular control mechanisms as a result, for example, of damage to their genetic code.

Although there is one single general mechanism of origin, neoplasia can evolve in a number of different ways and can manifest a huge range of symptoms. In all of them, however, an increase in the number of cancer cells is constant, due to the greater cell reproduction speed, on the basis of which a greater number of tumour cells multiplies and a lesser number dies, while those that survive continue to multiply and in some cases inhibit the growth of the physiologically normal cells. The growth of a tumour usually follows a geometrical law: it is very slow at the beginning but accelerates as the mass of the tumour increases. The critical dimension of a tumour is approximately 1 cubic centimetre: once it has reached this size, the tumour begins to grow very quickly, the first symptoms become apparent and it can be identified by medical examinations and analyses (tumoural markers in the blood), although the initial symptoms are often ignored or underestimated.

The high speed of reproduction of cancer cells also makes them more vulnerable to radiation than healthy tissue: this weakness is exploited to treat many types of solid tumour, for example by radiotherapy (bombardment with gamma rays), in an attempt to kill as many malignant cells as possible. Chemotherapy on the other hand exploits the specific sensitivity of the individual tumours to certain substances and currently, for each patient, a personalised mixture of several drugs is used. This "made-to-measure cocktail" almost always contains one or more mitosis inhibitors, such as taxol and its derivatives, to obstruct cell proliferation.

Although in some cases very effective, these therapies also have a high toxicity level in addition to significant side effects and are generally not well tolerated by patients.

It is therefore strongly necessary to find a form of treatment for said pathologies, which is not only effective due to its antineoplastic power but also advantageous due to reduced or null toxicity.

In addition to drugs, i.e. small molecules or substances and active ingredients of chemical derivation or obtained by synthesis, experimentation of new approaches aimed at treating neoplasia using progress in gene therapy, for example, and at developing treatments other than the above pharmacological and radiation therapy are increasingly numerous on the scientific and clinical scene.

All the therapeutic treatments currently known for neoplastic diseases have strengths and weaknesses, depending on the type of tumour to be treated, its stage and the organ affected.

The term apoptosis refers to a form of programmed cell death. In addition to its importance as a physiological biological phenomenon, it has acquired huge value from the medical point of view. In fact, faulty apoptosis processes are correlated with many diseases, for example excessive apoptotic activity can cause disorders deriving from loss of cells (see for example some neurodegenerative diseases, such as Parkinson's disease), while a defect in apoptosis activity can entail uncontrolled cell growth, which is the mechanism underlying neoplastic diseases.

The progress of scientific research into tumours field has permitted the identification of genes and proteins with anti-angiogenic or pro-apoptotic capabilities and consequently new ways of carrying what could be traditionally defined as the "active ingredient" directly to the site of the damage.

The most recently developed alternatives, like those described above, are therefore based on the possibility of developing new formulations, which permit the use of genes or proteins with anti-angiogenic or pro-apoptotic capabilities directly at the neoplastic site, in order to effectively combat the progress of the disease, at the same time limiting the side effects due to a more targeted and less generalised action. In other words, the aim is to direct the "active ingredients" directly into the neoplastic site, so that they perform their activity almost solely on the neoplastic cells and not also on the healthy cells of the organism, thus significantly limiting their toxicity.

Scientific progress and intense research in this sector have led to the identification of a protein called Apoptin, which performs many functions but in particular is able to re-activate some mechanisms of inhibition or promotion of apoptosis which are altered by the tumoural process and which induce a high level of resistance by the tumoural cells.

Apoptin is a viral protein of animal origin with antitumoural properties. The above mentioned protein, that is derived from chicken anemia virus (CAV), is able to induce apoptosis only in the tumoural cells or in those transformed.

Historically CAV, a small virus, has been identified as the aetiological agent of chicken infectious anemia. The CAV virion has no outer shell and has an average diameter of 23-25 nm. The capsid has an icosahedral structure with 32 capsomeres, consists of one single protein and contains circular DNA with one single strand of 2319 nucleotides. CAV has no hemagglutinant power. In the infected cell, the single-stranded genome replicates and transforms into double-strand DNA which, having one single activation point, is transcribed into one single linear mRNA. This mRNA contains, partially overlapping, three genes which encode the proteins VP1, VP2 and VP3, synthesised in the infected cell. VP1 is probably the capsidic protein, VP2 the one assigned to phosphorylation, having functions relating to constitution of the skeleton and assigned to assembly of the virus, while VP3 is the one with apoptotic properties.

In particular, the protein VP3 has been called apoptin because it has been found to cause cell death by apoptosis. In vitro studies have demonstrated said capabilities, and have highlighted its characteristic of forming non-covalent globular complexes, consisting of 30-40 subunits.

This protein consists of 121 amino acids and is rich in residues of proline, serine, threonine and basic amino acids. The basic region of the protein should permit interaction with the nucleic acid. The apoptin is active only on transforming cells, therefore tumoural and replicating cells and induced cells, and not on "healthy" physiologically replicating cells. For these reasons it appears to be an optimal target for anti-neoplastic treatment.

The term induced cells refers to those cells that do not "formally" have a neoplastic profile but which have assimilated and activated cell growth cascades for replication.

The protein apoptin is therefore particularly active but there are numerous limits to its use due to its conformational structure. Systems have been described in literature that permit the transport of apoptin, but none of them have proved to be truly effective. The most commonly used approach is gene therapy, i.e. the insertion of genetic material (in the form of DNA) inside the cells. The biggest limit of this therapy is the choice of the vector, of viral and non-viral origin, in which to insert the gene in question. If non-viral vectors are used for said purpose, they have the advantage of not being "problematic" vectors, since they are not of viral origin, but they are not suitable for containing particularly large quantities of DNA (in terms of Kb). The methods adopted to transfer the DNA without recourse to vectors of viral origin comprise: the injection of naked DNA, insertion by means of liposomes, insertion via the use of cationic polymers or bombardment by means of particles (gene gun).

Viral vectors on the other hand, since they are derived from viruses, are "capacious", i.e. suitable for containing large quantities of DNA, but are not as "safe" as the corresponding non-viral vectors. The use of viruses certainly has advantages, for example their aptitude for infecting numerous cell lines and their high efficiency in integration of the therapeutic gene in the genome. Their disadvantages are, for example, their intrinsic lability, which makes the procedure for their purification from the culture medium complex. Above all, however, the genome of viruses can be integrated and integration can be random, i.e. uncontrolled chromosome integration of the gene introduced into the cells could occur. For gene therapy, therefore, there are serious doubts concerning the lack of control over the destination of the genes transferred and the possibility of one or more cells being damaged. This can lead to the de-activation or activation of some genes different from the target genes, with the risk of insertional mutagenesis occurring.

Virus-like particles (VLPs) are known in the state of the art. The VLPs consist of viral proteins derived from structural proteins of a virus. In some cases these proteins are incorporated inside a lipidic bilayer. These particles similar to viruses have been obtained from the latter, but do not have viral nucleic acid, which means that they are not infectious. VLPs are in fact commonly used as a vaccine due to their intrinsic immunogenic properties.

### OBJECTS OF THE INVENTION

The object of the present invention is therefore to make available a method for the preparation of suitable carrier systems for pro-apoptotic proteins suitable for cellular recognition and their use in the treatment of neoplasia.

A further object of the invention is to make available suitable carrier systems for pro-apoptotic proteins which allow the "target" to be reached without being highly toxic.

### DISCLOSURE OF THE INVENTION

The present invention therefore concerns a method for the preparation of suitable carrier systems for pro-apoptotic proteins and accessory proteins suitable for cell recognition which comprises the development and preparation of lipidic particles able to express pro-apoptotic proteins and accessory proteins suitable for cell recognition.

The term "suitable carrier systems" refers to systems that permit expression of the apoptotic and accessory proteins. Said systems can be preferably lipidic particles of viral and non-viral origin, and even more preferably said systems are VLPs.

The term "proapoptotic proteins" refers to proteins that are able to activate directly or indirectly the mechanisms of cell death by apoptosis. In particular, it refers to the protein VP3, also called apoptin, of the CAV virus.

The term "accessory proteins" refers to the proteins suitable for carrying and anchoring to the neoplastic target cell for recognition. Alternatively, said accessory proteins can be those of the CAV, or VP1 and/or VP2 or all those suitable for the purpose.

Again according to the present invention, the term "accessory proteins" also refers to the possibility of simultaneous expression of both proteins suitable for carrying and anchoring to the neoplastic target cell for recognition and the proteins of the CAV virus, or VP1 and/or VP2 or all those suitable for the purpose.

A further subject of the present invention is therefore a carrier system which comprises use of the CAV virus, without its nucleic acid and modified in its cell anchoring coding sequence.

A further subject of the invention is the use of said lipidic particles in the treatment of neoplasia.

In general terms, the process subject of the invention involves the preparation, isolation and purification, as will be described in further detail below, of lipidic particles capable of expressing pro-apoptotic proteins and accessory proteins suitable for cell recognition in addition to their use in the treatment of neoplasia.

In particular the present invention concerns the process for preparing and purifying the lipidic particles expressing the pro-apoptotic proteins apoptins and those for cell recognition, said particles being for example Virus-Like Particles (VLPs) and said proteins for cell recognition being surface glycoproteins, for example glycoproteins for receptor-mediated endocytosis.

Any cell recognition system, also of the non-receptorial type, is comprised in the context of the present invention.

A further subject of the present invention is the use of said lipidic particles expressing the pro-apoptotic proteins and accessory proteins for the treatment of neoplasia.

Always according to the present invention, the use of said lipidic particles expressing the pro-apoptotic proteins and accessory proteins is directed also at the prevention of tumours, due to the fact that the apoptin can also discriminate induced cells and not only cells already transformed, and the treatment of metastases, which are generally formed by the same type of cell as the original tumour.

Again, the use of pro-apoptotic proteins according to the invention could be significant in the treatment of inflammatory conditions of autoimmune origin, for example rheumatoid arthritis.

The term "neoplasia" here refers to new cell formations, i.e. cells which have lost their physiological growth profile and are transformed or induced to grow.

As already mentioned, the present invention concerns the process for preparing, isolating and purifying the lipidic particles expressing said proteins.

A further subject of the present invention is the process for preparing and purifying the lipidic particles expressing the pro-apoptotic proteins and accessory proteins, said particles being for example Virus-Like Particles (VLPs) and said proteins for cell recognition being surface glycoproteins for receptor-mediated endocytosis.

A further subject of the present invention is the use of said lipidic particles expressing the pro-apoptotic proteins and those for cell recognition for the treatment of neoplasia.

It has been surprisingly found that VLPs prepared according to the invention are recruited to the neoplastic site, where they activate apoptotic mechanisms, inducing the death of the replicating tumour cells.

The process according to the invention therefore allows recruitment to the neoplastic site of the VLPs expressing the pro-apoptotic proteins and accessory proteins.

The present invention permits in particular the generation of modified VLPs, suitable for selective treatment of heterogeneous tumoural pathologies, i.e. it is possible according to the objects achieved by the present invention to obtain VLPs capable of recognising and "binding" via specific anchoring proteins modified according to the type of target cell and the site of the organ. In this way it is possible to obtain, for example, VLPs for neoplasia of hepatocytic origin, which will have as their anchoring accessory protein any protein able to recognise a receptor present on the hepatocytes.

The subject of the present invention is therefore a DNA sequence which encodes for the protein apoptin capable of efficiently forming a VLP. In particular the invention concerns expression plasmids which contain the DNA sequence that encodes for the apoptin and the sequence (or sequences) that encodes for the accessory proteins, for example for the proteins that anchor to the tumour site and/or the same proteins as the CAV, or VP1 and/or VP2 or all those suitable for the purpose.

Again according to the present invention, said expression plasmids that contain the DNA sequence which encodes for the apoptin can be different from those that contain the DNA sequence which encodes for the accessory proteins.

The present invention also concerns the host cells transformed by said expression plasmid(s), the method for production of the apoptin, the VLPs formed by said apoptin and the pharmaceutical compositions comprising said VLPs.

According to a preferred embodiment of the present invention, the plasmid is unique and encodes for a VLP of CAV, and for an accessory protein for example modified to recognise each time the cell receptor of the type of tumour cell to be treated.

In further detail, the process according to the invention comprises the following phases:
a) Prepare a recombinant expression vector
b) Insert said expression vector in a suitable host recombinant cell system
c) Produce apoptin and the cell recognition proteins capable of forming high efficiency VLPs
d) Purify the VLPs obtained according to the preceding phases

The process according to the invention therefore permits production and purification of a VLP that contains apoptin together with cell recognition accessory proteins which are capable of reaching and killing the neoplastic cells via apoptotic mechanisms.

The term "host recombinant cell system" here refers to prokaryote or eukaryote cells in which one or more molecules of encoding nucleic acid have been introduced, where said one or more molecules of nucleic acid encode for the protein apoptin or for the accessory proteins that form VLPs produced and purified according to the process of the invention.

The term "VLP" here refers to those VLPs obtained by a process which comprises at least one phase according to the recombinant DNA technology.

The VLPs obtained according to the process of the invention have high tropism for tumour cells.

In particular in the process according to the invention, said phase a) of preparation of the recombinant expression vector is performed so that the resulting plasmid contains the sequence of the protein apoptin and the sequence for at least one accessory protein as defined above.

According to said phase b), said suitable host recombinant cell system is prokaryote or eukaryote.

The phases a) and b) of the process described above are obtained by using the recombinant DNA techniques known in the state of the art, which comprise constructing the expression vector encoding for said proteins, transforming with said expression vector the host cell system and culturing the transformed cells in a suitable culture medium. Preferably the expression vector used according to the process of the invention also contains the sequences necessary for replication in the host cell system and at least one resistance gene which permits selection of the transformed cells. The host recombinant cell system is preferably any eukaryote or prokaryote cell line able to produce the protein apoptin. The host cell systems can comprise insect cells, mammal cells, vegetable cells, prokaryote cells and all those suitable for the purpose.

Phase c) comprises in particular the production of apoptin and accessory proteins capable of forming high-efficiency VLPs. Said process is conducted and adapted on the basis of the host cell system used and according to the knowledge of the state of the art.

Phase d) preferably comprises the phases of homogenising the host cells and purifying the homogenate by means of the techniques known in the state of the art.

The present invention furthermore concerns the pharmaceutical compositions containing said VLPs with high tropism and obtained according to the process subject of the present invention, together with any excipients known in pharmaceutical practice.

The present invention is better illustrated via the examples given below which are not limiting in any way.

### EXAMPLE 1 - construction of the recombinant vector

The vector baculovirus for the transfection was constructed using the plasmid creation kit, currently available on the market under the name Bac-to-Bac Baculovirus Expression System (Invitrogen). The cDNA encoding for the modified VLPs of CAV was inserted under the control of the citomegalovirus immediate early promoter (CMV-IE). The plasmid constructed as above containing the CAV sequence as deposited in the NCBI databank (GenBank Accession Number: M55918) was amplified by polymerase chain reaction (PCR). The plasmid contains additional sequences for restriction enzyme cut recognition. In this case adapter sequences were inserted for the restriction enzyme BamHI and also a stop codon. The resulting sequence formed as above was called pBacZint-A-CAV and was cloned in the vector pFastBac (Invitrogen), which also contains in its sequence the gene for resistance to ampicillin, following the manufacturer's protocol and using the cut recognition sites for the enzyme BamHI. This generated a recombinant vector pFastBacZint-A-CAV. The recombinant vector possesses the gene for resistance to ampicillin to permit selection of the cells containing the recombinant vector of interest. E. Coli cells were transformed with the vector pFastBacZint-A-CAV by electroporation. The E. Coli cells used for the transformation were previously made competent by using the One Shot TOP10 Competent Cell (Invitrogen) kit, currently available on the market. A bacmid called BacmidZint-A-CAV was isolated from the cells transformed with the recombinant vector pFastBacZint-A-CAV; said bacmid was subsequently purified and analysed to verify suitability according to common laboratory practice.

### EXAMPLE 2 - transfection of the pFastBacZint-A-CAV vector into the host cell system

The BacmidZint-A-CAV, generated as described in example 1, was then used for production of the P1 viral stock in Sf9 insect cells, according to the following procedure as described according to the manual provided by the manufacturer of the Bac-to-Bac Baculovirus Expression System (Invitrogen) kit. The manual was minimally adapted to requirements as follows:
1. Seed Sf9 cells in 6-well adhesive cell culture plates at a density of 9 x 10⁵ cells per well in a volume of 2 ml of Sf-900 II SFM growth medium. Add antibiotics to the medium: 2 ml of Sf-900 II SFM contains 50 units/ml of penicillin and 50 µg/ml of streptomycin.
2. Allow the cells to adhere at 27°C for at least 1 hour.
3. Prepare the Bacmid:Cellfectin Reagent complex as follows in 12x75 mm sterile tubes:
   dilute 1 µg of BacmidZint-A-CAV purified in 100 µl of non-supplemented Grace's medium;
   thoroughly mix the Cellfectin Reagent before use by inverting the tubes 5-10 times, remove 6 µl of Cellfectin Reagent and dilute it in 100 µl of non-supplemented Grace's Medium;
   combine the diluted BacmidZint-A-CAV with the diluted Cellfectin Reagent (total volume approx. 210 µl). Agitate and incubate for 15 - 45 minutes at ambient temperature.
4. While the DNA(BacmidZint-A-CAV):lipid (Cellfectin Reagent) complex is in incubation, remove the medium from the cells and wash once with 2 ml of non-supplemented Grace's Medium. Then remove the washing medium.
5. Add 0.8 ml of non-supplemented Grace's Medium to each tube containing the DNA (BacmidZint-A-CAV):lipid complex. Agitate gently and add the complex to each well containing the cells.
6. Incubate the cells in an incubator at 27°C for 5 hours.
7. Remove the DNA(BacmidZint-A-CAV):lipid complex and add 2 ml of complete growth medium (Sf-900 II SFM containing antibiotics) to the cells.
8. Incubate the cells in a humidified incubator at 27°C for 72 hours or until the signs of the viral infection are evident. Proceed with isolation of the P1 viral stock.

Once the cells of step 8 show signs of late infection (or after 72 hours of incubation), withdraw the medium containing the virus from each well (approx. 2 ml) and transfer everything to 15 ml snap-cap sterile test tubes. Centrifuge the test tubes at 500 x g for 5 minutes to remove cells and large debris. Transfer the clarified supernatant into 15 ml snap-cap sterile test tubes. These represent the P1 viral stock, to be kept at +4°C, protected from the light. Add fetal bovine serum at a final concentration of 2%, which acts as a substrate for the protease. For long-term storage, keep portions of viral stock at -80°C for subsequent re-amplification.

### EXAMPLE 3 - Generation of high-titer viral stock of BacmidZint-A-CAV

In order to optimise the viral stock generation protocol, as described in example 2, the following steps are performed:
- Prepare a suspension of Sf9 cells and place the cells on the plates at a cell density of 2 x 10⁶ cells/well. Incubate the cells at ambient temperature for 1 hour to allow adhesion.
- After 1 hour inspect the cells under an inverted microscope to verify adhesion.
- Add the P1 viral stock (i.e. stock as obtained from example 2) to each well.
- Incubate the cells for 48 hours in a humidified incubator at 27°C.
- At 48 hours post-infection, collect 2 ml of medium containing the virus from each well and transfer it into 15 ml snap-cap sterile test tubes. Centrifuge the test tubes at 500 x g for 5 minutes to remove cells and large debris.
- Transfer the supernatant to new 15 ml snap-cap test tubes. This is the P2 viral stock. Keep at +4°C protected from the light.
- Check that the viral titer of the new stock (P2) is not below 10⁸ pfu/ml.

### EXAMPLE 4 - Expression in eukaryote cells

The BHK mammal cells were cultured in DMEM supplemented with 10% of heat inactivated fetal bovine serum, 2 mM of L-glutamine and non-essential amino acids. The cells were kept in a humidified incubator at 37°C. The cells were then seeded on 6-well plates and incubated for one night. Before transfection, the cells were washed with Dulbecco's PBS (phosphate-buffered saline), followed by the addition of various volumes of solution containing the viruses. The PBS was then added to reach the final volume of 500 µl, and the plates were agitated on a rotating platform for 6 hours at 25°C. After the period of viral incubation, the solution containing BacmidZint-A-CAV virus was aspirated and the cells were washed again with PBS. The wells were filled with 2 ml of complete medium and incubated at 37°C. The cells were collected 2 days after transduction and lysed in a lysis buffer (1% NP-40, 1% Triton X-100, 0.5% sodium desoxycholate, 1 µg ml-1 aprotinin, 1 mM phenylmethylsulphonyl fluoride, 10 mM Tris, 1 mM EDTA, pH 7.4), on ice for 30 minutes. They were then stratified on sucrose cushions 20% and ultracentrifuged at 38000 rpm (P40ST rotor, Hitachi) for 5h.

At the end of the process the proteins can be purified according to the known techniques.

## Claims

1. Process for obtaining VLPs (Virus-Like Particles) expressing the pro-apoptotic protein apoptin and at least one accessory protein, which comprises the following phases:
a) Prepare at least one recombinant expression vector
b) Insert said expression vector in at least one suitable host recombinant cell system
c) Produce apoptin and said at least one accessory protein, obtaining said VLPs
d) Purify said VLPs obtained according to the preceding phases

2. Process as claimed in claim 1 **characterised in that** said phase a) comprises the preparation of a recombinant expression vector which contains the gene of the protein apoptin and of a recombinant expression vector which contains the gene of at least one accessory protein for cell recognition.

3. Process as claimed in claim 1 **characterised in that** said phase b) comprises the insertion of said expression vector in a host recombinant cell system, wherein said host cell system is prokaryote or eukaryote.

4. VLPs (Virus-Like Particles) expressing the pro-apoptotic protein apoptin and at least one accessory protein.

5. Pharmaceutical composition containing as active ingredient VLPs expressing the pro-apoptotic protein apoptin and at least one accessory protein.

6. Pharmaceutical composition as claimed in claim 4 in the form of injectable formulations.

7. Use of VLPs expressing the pro-apoptotic protein apoptin and at least one accessory protein for the preparation of a medicament for the treatment of neoplasia.

8. Use of VLPs expressing the pro-apoptotic protein apoptin and at least one accessory protein for the prevention of neoplasia.

9. Use of VLPs expressing the pro-apoptotic protein apoptin and at least one accessory protein for the treatment of neoplasia metastases.

10. VLPs expressing the pro-apoptotic protein apoptin and at least one accessory protein for use as a medicament.

11. VLPs expressing the pro-apoptotic protein apoptin and at least one accessory protein for use as a medicament for the treatment of neoplasia.
